(19) Europäisches Patentamt
European Patent Office
Office européen des brevets

(11) Veröffentlichungsnummer: **0 031 932**
**B1**

(12) # EUROPÄISCHE PATENTSCHRIFT

(45) Veröffentlichungstag der Patentschrift: **01.07.87**

(21) Anmeldenummer: **80107955.9**

(22) Anmeldetag: **17.12.80**

(51) Int. Cl.⁴: **C 07 D 309/32**, C 07 D 309/30, C 07 D 303/38, C 07 C 69/63, C 07 C 69/708, C 07 C 59/135, C 07 C 57/52

(54) **Substituierte Lactone, Pentacarbonsäurederivate und Verfahren zu ihrer Herstellung.**

(30) Priorität: **22.12.79 DE 2952068**
**03.12.80 DE 3045555**
**06.12.80 DE 3046059**

(43) Veröffentlichungstag der Anmeldung: **15.07.81 Patentblatt 81/28**

(45) Bekanntmachung des Hinweises auf die Patenterteilung: **01.07.87 Patentblatt 87/27**

(84) Benannte Vertragsstaaten: **BE CH DE FR GB IT LI NL SE**

(56) Entgegenhaltungen:
**DE-A-860 203**
**US-A-2 476 668**

**C.R. ACAD. Sc. Paris, Série C, t 284, 21. Februar 1977, Montreuil, Fr. D. PLOUIN et al.: "Composés mono et diacétyléniques. Réactivité en milieu basique d'esters Beta-cétoniques gamma-acétyléniques vrais"**
**CHEMICAL ABSTRACTS, Band 72, 1970, Seite 417, Zusammenfassung 43304f Columbus, Ohio, U.S.A. E.G. MESROPYRAN et al.: "Synthesis and reactions of diethyl esters of alkylglycidylmalonic acids"**
**CHEMICAL ABSTRACTS, Band 78, 1973, Seite 451, Zusammenfassung 124222d Columbus, Ohio, U.S.A. V. ARAKELYAN et al.: "Alpha-Alkyl-6-(aryloxy)-gamma-valerolactones"**
**JOURNAL OF ORGANIC CHEMISTRY, Band 41, Nr. 5, 5. März 1976, Seiten 885-887 Washington, U.S.A.**

(73) Patentinhaber: **DYNAMIT NOBEL AKTIENGESELLSCHAFT, Postfach 1209, D-5210 Troisdorf, Bez. Köln (DE)**

(72) Erfinder: **Schmidt, Hans- Georg, Dr., Porzer Strasse 15, D-5216 Niederkassel 3 (DE)**

(56) Entgegenhaltungen: (Fortsetzung)
**J.H. BABLER et al.: "Regioselectivity in the cyclization of beta, gamma-epoxy carbanions. Application to the total synthesis of transchrysanthemic acid"**
**CHEMICAL ABSTRACTS, Band 92, Nr. 1, 7. Januar 1980, Seite 171, Nr. 1737g Columbus, Ohio, U.S.A. P. ORTIZ DE MONTELLANO et al.: "Destruction of cytochrome P-450 by 2-isopropyl-4-pentenamide and methyl 2-isopropyl-4-pentenoate: mass spectrometric characterization of prosthetic heme adducts and nonparticipation of epoxide metabolites"**
**CHEMICAL ABSTRACTS, Band 55, Nr. 5, 6. März 1961, Spalte 4358d Columbus, Ohio, U.S.A. Y. KAWAI: "Synthesis of glutaric acid derivatives from cyclopentadiene. II. Decomposition products of 3-chlorocyclopentene ozonide"**
**JOURNAL OF THE CHEMICAL SOCIETY, 1949, Seiten 244-248 London, G.B. R.M. EVANS et al.: "Dithiols. Part II. 2:3-Dimercaptopropyl ethers of glycerol and of glycollic acid, with some further observations on "BAL-Intrav"**
**Journal of Organic Chemistry, Vol. 41, No. 5, 1976, S. 886**

**Die Akte enthält technische Angaben, die nach dem Eingang der Anmeldung eingereicht wurden**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

EP 0 031 932 B1

**Beschreibung**

Die vorliegende Erfindung behandelt neue Lactone und Pentancarbonsäurederivate, die zur Herstellung von Cyclopropancarbonsäureestern dienen. Ebenfalls behandelt die Erfindung Verfahren zur Herstellung dieser neuen Verbindungen.

Substituierte Acetale von Formelcyclopropancarbonsäureestern der allgemeinen Formel I

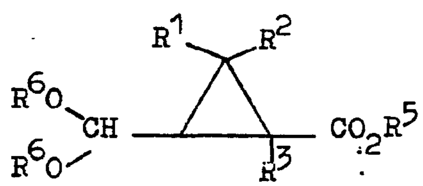

I,

in der $R^1$ $R^2$ $R^3$ für Wasserstoff oder $C_1$ bis $C_{10}$ Alkylgruppen und $R^5$ und $R^6$ für $C_1$ bis $C_6$ Alkylgruppen stehen, wobei mindestens einer der Reste $R^1$ oder $R^2$ eine Alkylgruppe ist, sind wichtige Zwischenprodukte zur Herstellung von Insektiziden der Pyrethroidklasse (vgl. Synthetic Pyrethroids, ACS Symposium Serie 42, American Chemical Society Washington D.C. 1977). Die Herstellung von Verbindungen der Formel I ist bereits in der DE-OS 26 15 160 beschrieben. Die Synthese erfolgt dort durch Umsetzung von Triphenylisopropylphosphoniumjodid mit 4-Oxo-butensäurealkylester unter Einsatz stöchiometrischer Mengen Butyllithium. Diese Ausgangsprodukte sind schwierig zugänglich, und insbesondere die metallorganische Verbindung Butyllithium ist leicht entzündlich und deshalb nur mit großem Aufwand handhabbar.

Es bestand deshalb die Aufgabe, neue Wege zur Herstellung der Cyclopropancarbonsäureester aufzufinden, die die genannten Nachteile nicht zeigen.

In Erfüllung dieser Aufgabe wurden nun neue Verbindungen der Formel II gefunden

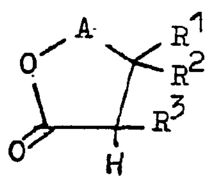

II,

in der A für $-\overset{|}{C}H-CH_2-X$ (X = Cl, Br oder OH)

$-CHY-CHY-$ (Y = Cl, Br)

oder $-\underset{H}{\overset{}{C}}=\underset{H}{\overset{}{C}}-$

steht und $R^1$, $R^2$ und $R^3$ gleiche oder verschiedene Reste aus der Gruppe Wasserstoff und $C_1$ bis $C_{10}$ Alkyl bedeuten, wobei mindestens einer der Reste $R^1$ oder $R^2$ ein solcher Alkylrest ist, beschrieben werden.

Lactone mit diesem speziellen Substitutionsmuster sind noch nicht beschrieben. Lediglich über Lactone, bei denen A für $-CH-CH_2X$ steht, sind Literaturstellen bekannt (DE-PS 860 203 und Chemical Abstracts, Bd. 76 (1970) Seite 417, 43304 f.). Es werden jedoch nur Lactone mit X = Br bzw. X = OH beschrieben, für die $R^1$ = $R^2$ = H steht.

Diese Lactone sind deshalb für Pyrethroide nicht als Zwischenprodukt einsetzbar, da in den Pyrethroiden mindestens eine der Gruppen $R^1$ oder $R^2$ eine Alkylgruppe sein muß.

In Erfüllung der vorgenannten Aufgabe wurden als weitere neue Verbindungen, die zur Herstellung der Cyclopropanverbindung notwendig sind, die Pentensäureester der Formel III

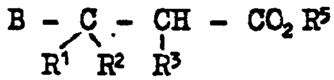 III

gefunden, wobei $R^1$, $R^2$, $R^3$ die obige Bedeutung haben, ebenfalls mit der Einschränkung, daß mindestens einer der Reste $R^1$ und $R^2$ eine Alkylgruppe ist, und

B für

B für 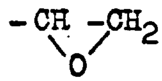 ; 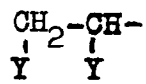

oder 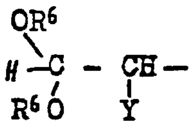 (Y = Cl oder Br)

und $R^5$, $R^6$ für einen niedrigen $C_1$ bis $C_6$ Alkylrest stehen.

Die Verbindungen der Formel III mit

B = 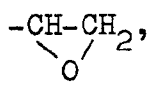, $(R^6O)_2CH-CHY-$

werden aus den Verbindungen der Formel II hergestellt und zu Verbindungen der Formel I weiterverarbeitet.

Die Verbindungen der Formel III mit B = $CH_2Y$-CHY- werden zur Herstellung der Verbindungen der Formel II mit A = -CH-$CH_2Y$ verwendet. Beispiele der Verbindungen III mit B = $CH_2Y$-CHY- sind in der Literatur beschrieben (DE-PS 860 203), jedoch besitzen die dort beschriebenen Verbindungen nicht das geforderte Substitutionsmuster.

Die Acetale des Anspruchs 12, bei denen der Rest B für

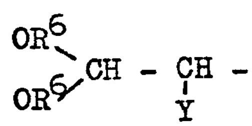

steht, werden erhalten wenn man die Verbindungen des Anspruchs 1, bei denen A für

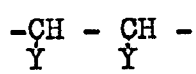

steht, mit Alkali- oder Erdalkalialkoholaten in Gegenwart überstöchiometrischer Mengen von aliphatischen Alkoholen mit 1 bis 10 C-Atomen umsetzt. Bei dieser Umsetzung ist das bevorzugte Alkoholat Natriumalkoholat. Die Alkoholmenge kann zwischen einem 2- bis 200fachen molaren Überschuß variiert werden. Die Reaktionstemperatur liegt im allgemeinen zwischen +10 und +60°C.

Die Isolierung der bei dieser Umsetzung erhaltenen Acetale erfolgt durch fraktionierte Destillation. Wenn kein Überschuß an Alkoholat eingesetzt ist, kann diese Destillation direkt anschließend an die Umsetzung durchgeführt werden. Bei Anwesenheit überschüssigen Alkoholats ist es jedoch empfehlenswert, dieses durch Extraktion mit Wasser zu entfernen und anschließend das Rohprodukt destillativ aufzuarbeiten.

Bei der Herstellung der Verbindungen II, in denen A für

$$-CH-CH_2-Halogen$$

steht, werden Pentensäureester der allgemeinen Formel

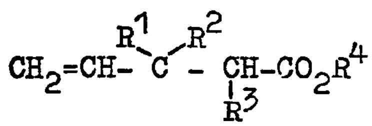 ,

in der $R^1$, $R^2$, $R^3$ für die genannten Alkylreste oder für H steht, wobei mindestens einer der drei Reste ein Alkylrest ist, $R^4$ für einen Alkylrest mit 1 bis 4 C-Atomen steht, zuerst mit Halogen zu einer Verbindung der allgemeinen Formel

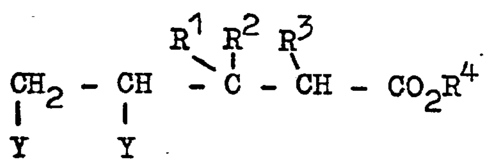 III b

(Y = Halogen) umgesetzt. Anschließend wird das erhaltene Additionsprodukt erhitzt, wobei Lactone der allgemeinen Formel

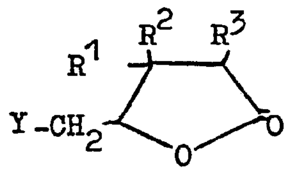 II a

entstehen.

Die Halogenaddition an den Pentensäureester wird in an sich bekannter Weise in einem organischen Lösungsmittel vorzugsweise in einem flüssigen Chlorkohlenwasserstoff, wie z. B. $CCl_4$, durch Zudosieren des Halogens bei Temperaturen zwischen -10 bis +40°C vorgenommen. Das Lösungsmittel wird nach Beendigung der Reaktion durch Destillation entfernt.

Die Ringbildung zu den Lactonen der Formel II b erfolgt bei Temperaturen zwischen 150 und 200°C. Es kann sowohl ohne als auch mit Lösungsmittel (z. B. Dekalin) gearbeitet werden. Während des Erhitzens entsteht ein Leichtsieder, der vorzugsweise sofort destillativ aus dem Reaktionsgemisch entfernt wird. Eine zweckmäßige Verfahrensweise besteht darin, das Halogenadditionsprodukt II a ohne Isolierung zu erhitzen.

Für die Herstellung von Lactonen der Formel I, in denen A =

$$-CH-CH_2OH$$

und $R^1$, $R^2$ und $R^3$ beliebige Reste aus der Gruppe $C_1$- bis $C_{10}$-Alkyl oder H sein können, werden Lactone der Formel I, in denen A =

$$-CH-CH_2Hal$$

ist, zuerst mit basischen Mitteln zu Verbindungen der allgemeinen Formel III

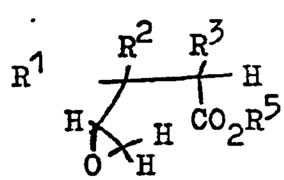 III

in der $R^1$, $R^2$, $R^3$ beliebige Reste aus der Gruppe $C_1$- bis $C_{10}$- Alkyl oder H sein können und $R^5$ für einen Alkylrest mit 1 bis 4 C-Atomen, Wasserstoff oder ein Alkali- oder Erdalkalimetall steht, umgesetzt. Anschließend erfolgt die Lactonbildung durch Behandeln mit Säuren.

Die Behandlung mit den basischen Mitteln, die zur zur Bildung des Epoxids führt, wird bei Temperaturen zwischen -10 und +50°C vorgenommen. Als basische Mittel werden Alkali- oder Erdalkalialkoholate,

-carbonate oder -hydroxide eingesetzt. Vorzugsweise werden Lösungsmittel wie Alkohole, Äther oder Wasser verwendet. Die Basen werden stöchiometrisch oder im Überschuß eingesetzt.

Wenn diese Umsetzung mit Alkoholaten als basischem Mittel in Alkoholen durchgeführt wird, werden die Pentansäureester erhalten. Man wählt vorzugsweise einen Alkohol, der der Alkoholkomponente des Alkoholats entspricht. Diese enthält vorzugsweise zwischen 1 und 4 C-Atomen.

Aus den dabei direkt erhaltenen Epoxiden werden die Lactone der Formel I mit A =

$$-\underset{|}{C}H-CH_2OH$$

durch Einrühren des Epoxids der Formel III in ein saures Medium, wie z. B. verdünnte wässrige Mineralsäuren oder verdünnte organische Säuren hergestellt. Die Säuren werden bevorzugt in einem Lösungsmittel eingesetzt. Dieses kann außer dem bevorzugt eingesetzten Wasser auch ein organisches Lösungsmittel, wie Alkohole, Äther, Eisessig, Tetrachlorkohlenstoff und dergleichen sein.

Derartige Umsetzungen von Epoxiden zu γ-Hydroximethyl-γ-butyrolacetonen sind bereits beschrieben (Chemical Abstracts 72 (1970) 43304 f.). Allerdings zeigen die dort auf geführten Epoxide gravierende Unterschiede in den Substituenten. Außerdem ist für die Umwandlung ein Erhitzen auf 80 bis 85° C notwendig. Die vorliegende Erfindung hebt sich von diesem Stand der Technik dadurch ab, daß die Reaktion bereits bei Zimmertemperatur erfolgt.

Die Säuren wirken bereits in katalytischen Mengen, können jedoch gegebenenfalls im größeren Überschuß eingesetzt werden. Die bevorzugten Mengen liegen zwischen 0,01 und 10 Mol-%.

Die Reaktionstemperatur kann in weiten Bereichen (-20 bis +200° C) gewählt werden.

Als Säuren eignen sich sowohl Lewis- als auch Broensted-Säuren, wie z. B. $AlCl_3$, $BF_3$, $H_sSO_4$, $H_3PO_4$, $HNO_3$ und organische Carbonsäuren.

Eine zweckmäßige Verfahrensweise besteht darin, das gebildete Epoxid ohne Isolierung sofort mit der Säure zu behandeln.

Die Herstellung der Lactone der Formel II, bei denen A = -CH=CH- ist, erfolgt durch Thermolyse von Lactonen der Formel I, bei denen A =

$$-\underset{|}{C}H-CH_2OH$$

ist. Sie wird bevorzugt im Temperaturbereich zwischen +200 bis +500° C ausgeführt.

Zweckmäigerweise tropft man die Ausgangsverbindung durch ein erhitztes, mit temperaturbeständigem Material, z. B. keramischem Material, gefülltes Rohr. Die Thermolyse wird bevorzugt in einer Inertgasatmosphäre durchgeführt.

Die Ausgangsverbindungen können dabei in Substanz oder in Lösungen eingesetzt werden. Als Lösungsmittel kommen temperaturstabile Verbindungen wie flüssige aromatische Kohlenwasserstoffe, z. B. Benzol, Toluol, Xylol, bevorzugt in Frage. Die Reaktion erfolgt unter Wasserabspaltung, wobei das Wasser bei Verwendung von mit Wasser nicht mischbaren Lösungsmitteln vor der destillativen Aufarbeitung abgeschieden werden kann.

Verbindungen der allgemeinen Formel II, bei denen der Rest A für

$$-\underset{\underset{Y}{|}}{C}H - \underset{\underset{Y}{|}}{C}H -$$

steht, werden in an sich bekannter Weise aus Verbindungen der Formel II hergestellt, bei denen der Rest A für -CH=CH- steht, indem man an diese Verbindungen Halogen addiert. Diese Addition erfolgt vorzugsweise in einem flüssigen Chlorkohlenwasserstoff, wie z. B. Tetrachlorkohlenstoff. Auch andere inerte Lösungsmittel, wie z. B. Pentan, sind geeignet. Die Reaktionstemperatur sollte dabei nach Möglichkeit +40°C nicht überschreiten. Im allgemeinen liegt sie zwischen -50 und +40°C. Nach beendeter Addition wird das Lösungsmittel destillativ entfernt. Im Rückstand fällt das gewünschte Additionsprodukt in einer solchen Reinheit aus, die seine direkte Weiterverarbeitung erlaubt. Gegebenenfalls kann es umkristallisiert werden.

Dieses Dihalogenvalerolacton ist wiederum ein Zwischenprodukt für die bereits oben genannten Acetale, die der Formel des Anspruchs 12 entsprechen, in denen der Rest B für

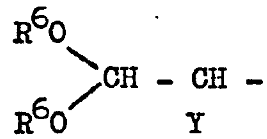

steht.

Die Umsetzung der Acetale der Formel III

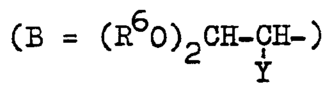

zu den Verbindungen der allgemeinen Formel I erfolgt mit Hilfe von Alkoholaten.

Die oben genannten alkylsubstituierten Penten-4-carbonsäureester sind auf einfache Weise durch Umsetzung von entsprechend substituierten Allylalkoholen mit entsprechenden substituierten Orthoessigsäurealkylestern zugänglich (Tetrahedron Letters, 1977, Seite 2543).

Die neuen Lactone der allgemeinen Formel I stellen wertvolle organische Zwischenprodukte zur Herstellung von Pflanzenschutzmitteln, Pharmazeutica und Farbstoffen dar. Sie eignen sich besonders zur Herstellung von substituierten Cyclopropancarbonsäurederivaten, die als Vorprodukte für Insektizide bereits bekannt sind, und die bisher nur auf anderem Wege zugänglich waren. Zur Herstellung dieser Verbindungen ist es nur notwendig, Pentancarbonsäurederivate mit der endständigen Acetalgruppierung mit Alkoholaten auf an sich bekannte Weise umzusetzen.

**Beispiel 1**

12,7 g 3,3-Dimethyl-penten-4-säuremethylester werden in 18,9 ml Tetrachlorkohlenstoff gelöst und zu dieser Mischung 14,3 g Brom in 44,9 ml Tetrachlorkohlenstoff bei 10 bis 20°C zugetropft. Nach Entfernen des Lösungsmittels im Vakuum erhält man den 3,3-Dimethyl-4,5-dibrom-pentan-säuremethylester in nahezu quantitativer Ausbeute.

NMR-Spektrum (30 MHZ, $CCl_4$): $\delta$ = 1,1 (s, 3 H); 1,3 (s, 3 H); 2,4 (s, 2 H); 2,7 (s, 3H); 3,3-4,7 (m, 3 H).

**Beispiel 2**

20,0 g 3,3-Dimethyl-4,5-dibrom-pentansäuremethylester werden in einer Apparatur für eine fraktionierende Destillation bei einem Druck von 106 Pa 30 min am Rückfluß gekocht und dann fraktioniert destilliert. Bei 95 bis 96°C gehen 11,3 g Dihydro-4,4-dimethyl-5-brommethyl-(3 H)-furanon über.

NMR-Spektrum (100 MHZ, $CCl_4$): $\delta$ = 1,10 (s, 3 H); 1,26 (s, 3 H); 2,41 (m, 2 H); 3,55 (m, 2 H); 4,37 (m, 1 H).

**Beispiel 3**

43,7 g Dihydro-4,4-dimethyl-5-brommethyl-(3 H)-furanon werden in 166 ml Methanol gelöst und diese Mischung nach Zugabe von 11,4 g Natriummethylat 5 Std. am Rückfluß gekocht. Das Methanol wird im Vakuum entfernt und der Rückstand in Wasser aufgenommen. Die wässrige Lösung wird ausgeäthert und die organische Phase über Molekularsieb getrocknet und fraktioniert destilliert. Bei einem Druck von 1 729 Pa gehen bei 86 bis 88°C 29,5 g 4,5-Epoxi-3,3-dimethyl-pentansäuremethylester über.

NMR-Spektrum (100 MHZ, $CCl_4$): $\delta$ = 0,95 (s, 3 H); 0,98 (s, 3 H); 2,22 (m, 2 H); 2,52 (m, 2 H); 2,77 (m, 1 H); 3,60 (m, 3 H).

**Beispiel 4**

21,1 g 4,5-Epoxi-3,3-dimethyl-pentansäuremethylester werden in 50 ml $CCl_4$ gelöst und diese Mischung in 12 ml 2n $H_2SO_4$ 5 Std. geschüttelt. Danach wird die organische Phase abgetrennt, die wässrige Phase mit Äther extrahiert und die vereinigten organischen Phasen über Molekularsieb getrocknet. Nach Entrfernen des organischen Lösungsmittels bleibt ein kristalliner Rückstand (17,6 g), der als Dihydro-4,4-dimethyl-5-hydroximethyl-(3 H)-furanon (Fp. 40-42°C) identifiziert wird.

NMR-Spektrum (100 MHZ, $CDCl_3$): δ = 1,08 (s, 3 H); 1,17 (s, 3 H); 2,37 (m, 2 H); 3,77 (m, 2 H); 4,15 (m, 1 H); 3,80 (bs, 1 H).

**Beispiel 5**

2 g Dihydro-4,4-dimethyl-5-hydroxi-methyl-(3 H)-furanon werden in 20 ml Toluol gelöst und diese Mischung unter Stickstoff durch ein beheizbares Quarzrohr (Länge 30 cm, Durchmesser 2,2 cm) welches mit Quarzkugeln gefüllt ist, bei einer Temperatur von 300° innerhalb 80 min durchgetropft und in einer gekühlten Vorlage aufgefangen. Das Toluol wird bei Normaldruch durch fraktionierende Destillation entfernt. Der Rückstand wird bei 1 596 Pa destilliert. Es werden 0,5 g 3,4-Dihydro-4,4-dimethyl-α-pyron erhalten (Sdp. 1 596 Pa: 60-62°C). Darüber hinaus werden 1,3 g Ausgangsprodukt zurückgewonnen.

NMR-Spektrum (100 MHZ, $CCl_4$) δ ppm: 1,12 (s, 6 H); 2,40 (bs, 2 H); 5,11 (d, 1 H); 6,37 (d, 1 H).

**Beispiel 6**

10 g Dihydro-5-brommethyl-(3 H)-furanon werden mit 3,0 g Natriummethylat in 37 ml Methanol 5 Stunden am Rückfluß gekocht. Das Lösungsmittel wierd im Vakuum entfernt, der Rückstand in 20 ml $H_2O$ aufgenommen, dieses mit Äther extrahiert, die ätherische Phase über Molekularsieb getrocknet und der Äther im Vakuum entfernt. Der Rückstand wird bei 20 nm destilliert. Man erhält 6,4 g 4,5-Epoxipentancarbonsäuremethylester (Sdp: 85°C / 2 660 Pa.)

**Beispiel 7**

6 g 4,5-Epoxipentansäuremethylester werden in einer Mischung aus 10 ml Äther und 10 ml 2nHCl 1 Stunde geschüttelt. Danach wird der Äther abgetrennt und die wässrige Phase mit Äther perforiert. Aus den vereinigten getrockneten ätherischen Phasen erhält man nach Entfernung des Äthers im Vakuum 3,7 g Dihydro-5-hydroximethyl-(3 H)-furanon.

**Beispiel 8**

8 g Dihydro-5-hydroximethyl-(3 H)-furanon werden durch ein beheizbares Quarzrohr (Länge 30 cm, Durchmesser 2,2 cm), welches mit Montmorillonit-Kugeln gefüllt ist, bei einer Temperatur von 300°C innerhalb 80 min durchgetropft und in einer gekühlten Vorlage aufgefangen. Man erhält 5,9 g 3,4-Dihydropyron.

H-NMR-Spektrum (30 MHZ): δ ppm = 2,0 bis 3,0 (m, 4 H); 5,40 (m, 1 H); 6,65 (m, 1 H).

**Beispiel 9**

17,6 g 4,4-Dimethyl-3,4-dihydro-α-pyron werden in 45 g $CCl_4$ gelöst, und zu dieser Mischung werden unter Rühren 22,3 g Brom in 59 g $CCl_4$, gelöst bei 5 bis 10°C, zugetropft. Nach dem Zutropfen wird das Lösungsmittel im Wasserstrahlvakuum entfernt. Man erhält 39,5 g kristallines 4,4-Dimethyl-5,6-dibrom-δ-valerolacton. (Fp. 85 bis 87°C aus $CCl_4$). $^1$H-NMR-Spektrum (100 MHZ, $CCl_4$): $\delta_{ppm}$ = 1,16 (s, 3 H); 1,24 (s, 3 H); 2,70 (m, 2 H); Zentren bei 4,32 und 4,41 (m, 1 H); Zentren bei 6,46 bis 6,61 (m, 1 H)

**Beispiel 10**

9,5 g 4,4-Dimethyl-5,6-dibrom δ-valerolacton werden in 5 g Methanol gelöst und zu dieser Mischung 1,67 g Na-Methylat, in 11 g Methanol gelöst, innerhalb 2 Stunden unter Rühren bei 20 bis 30°C zugetropft. Anschließend gießt man die Reaktionsmischung in 50 ml $H_2O$ und extrahiert dies mit Methylenchlorid. Nach Trocknen der organischen Phase über ein Molekularsieb und Entfernen des Lösungsmittels im Wasserstrahlvakuum erhält man 7,8 g 5,5-Dimethoxi-4-brom-3,3-dimethylpentansäuremethylester.

H-NMR-Spektrum (30 MHZ, $CCl_4$): $\delta_{ppm}$ = 1,20 (s, 6 H); 2,53 (s, 2 H); 3,45 (s, 6 H); 4,48 (s, 2 H).

**Beispiel 11**

6,2 g 5,5-Dimethoxi-4-brom-3,3-dimethylpentancarbonsäuremethylester werden in 3,3 g Methanol gelöst und zu dieser Mischung eine Lösung aus 1,3 g Na-Methylat und 8,2 g Methanol zugetropft. Danach wird 15 Stunden bei 50°C gerührt. Anschließend wird die Reaktionsmischung auf Wasser gegeben, mit Diäthyläther extrahiert und die organische Phase getrocknet. Nach Entfernen des Äthers im Wasserstrahlvakuum wird der Rückstand destilliert. Man erhält 3,55 g cis, trans-2,2-Dimethyl-3-formyl-(dimethyl-acetal)-cyclopropancarbonsäuremethylester.

**Beispiel 12**

0,5 g 4,4-Dimethyl-3,4-dihydro-α-pyron werden in 4,5 g Tetrachlormethan gelöst, und in diese Mischung werden 0,3 g gasförmiges Chlor bei 10°C eingeleitet. Nach Entfernen des Lösungsmittels im Wasserstrahlvakuum erhält man 0,7 g cis, trans-4,4-Dimethyl-5,6-dichlor-δ-valerolacton.

H-NMR-Spektrum (30 MHZ, $CCl_4$): $\delta_{ppm}$ = 1,25 (bs, 6 H); 2,70 (bs, 2 H); 4,15; 4,20 (2 d, 1 H); 6,15-6,45 (2 d,1 H).

**Beispiel 13**

166 g 4,4-Dimethyl-5,6-dichlorvalerolacton werden in 124 g Methanol bei Raumtemperatur gelöst, und zu dieser Lösung wird eine methanolische Natriummethylat-Lösung (48 g $NaOCH_3$ in 247 g Methanol) getropft. Es wird 1 Stunde bei Raumtemperatur nachgerührt. Das Methanol wird im Wasserstrahlvakuum entfernt und der Rückstand in kleinen Portionen im Hochvakuum destilliert. Man erhält 187,8 g 5,5-Dimethoxi-4-chlor-3,3-dimethylpentansäuremethylester (Siedepunkt 13,3 Pa: 117 bis 119°C).

H-NMR-Spektrum (80 MHZ, $CDCl_3$): δ ppm = 1,19 (s, 6 H); 2,51 (m, 2 H); 3,43 (s, 3 H); 3,44 (s, 3 H); 3,66 (s, 3 H); 4,24 (d, 1 H); 4,50 (d, 1 H).

**Beispiel 14**

8 g 5,5-Dimethoxi-4-chlor-3,3-dimethylpentancarbonsäuremethylester werden in 30 ml Diäthylenglycoldimethyläther mit 2,2 g $NaOCH_3$ 5 Stunden bei 110°C gerührt. Das entstandene Kochsalz wird abfiltriert, mit Diäthyläther gewaschen und die organischen Phasen werden vereinigt. Nach Entfernung des Diäthyläthers im Vakuum wird der Diäthylenglycoläther im Wasserstrahlvakuum abdestilliert und der Rückstand im Hochvakuum destilliert. Man erhält 5,2 g 2,2-Dimethyl-3-formyl-(dimethylacetal)-cyclopropancarbonsäuremethylester.

**Patentansprüche**

1. Substituierte Lactone der Formel

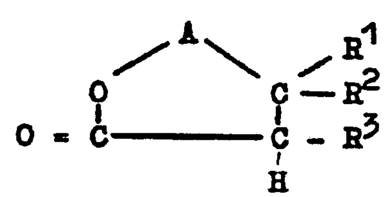

in der A für

(X = Cl, Br oder OH), -CHY-CHY-(Y = Cl oder Br) oder

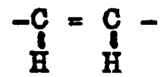

steht und $R^1$, $R^2$ und $R^3$ gleiche oder verschiedene Reste aus der Gruppe Wasserstoff und $C_1$ bis $C_{10}$-Alkyl bedeuten, wobei mindestens einer der Reste $R^1$ oder $R^2$ ein solcher Alkylrest ist.

2. Verfahren zur Herstellung von Lactonen gemäß Anspruch 1, bei denen der Rest A für

$$-CH-CH_2Cl \text{ oder } -CH-CH_2Br$$

steht, dadurch gekennzeichnet, daß man Pentensäureester der Formel

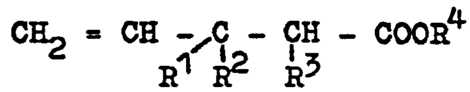

in der $R^1$, $R^2$ und $R^3$ = die obengenannte Bedeutung haben mit Chlor oder Brom bei Temperaturen zwischen -10 und +40°C umsetzt und das dabei erhaltenen Additionsprodukt auf Temperaturen zwischen 150 und 200°C erhitzt.

3. Verfahren gemäß Anspruch 2, dadurch gekennzeichnet, daß die Additionsreaktion in einem flüssigen Chlorkohlenwasserstoff als Lösungsmittel durchgeführt wird.

4. Verfahren zur Herstellung von Lactonen gemäß Anspruch 1, bei denen der Rest A für

$$-CH-CH_2OH$$

steht, dadurch gekennzeichnet, daß man Lactone gemäß Anspruch 1, bei denen der Rest A gleich

$$-CH-CH_2Br$$

oder

$$-CH-CH_2Cl$$

ist, mit basischen Mitteln zu Pentansäurederivaten der allgemeinen Formel

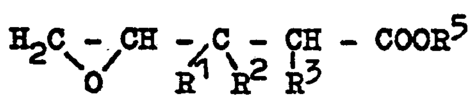

umsetzt, wobei $R^5$ für einen niederen Alkylrest mit 1 bis 4 C-Atomen, Wasserstoff oder für ein Alkali- oder Erdalkalimetall steht, und anschließend die Lactonbildung durch Behandlung mit Säuren durchführt.

5. Verfahren gemäß Anspruch 4, dadurch gekennzeichnet, daß als basische Mittel Alkali- oder Erdalkalialkoholate von Alkoholen mit 1 his 4 C-Atomen eingesetzt werden.

6. Verfahren gemäß Anspruch 4, dadurch gekennzeichnet, daß als basisches Mittel ein Alkali- oder Erdalkalihydroxyd oder -carbonat eingesetzt wird.

7. Verfahren gemäß einem der Ansprüche 4 bis 6, dadurch gekennzeichnet, daß die Lactonbildung mit Hilfe von Mineralsäuren oder organischen Säuren durchgeführt wird.

8. Verfahren gemäß einem der Ansprüche 4 bis 6, dadurch gekennzeichnet, daß die Lactonbildung mit Hilfe von Lewis-Säuren durchgeführt wird.

9. Verfahren zur Herstellung von Lactonen gemäß Anspruch 1, bei denen A für -CH=CH- steht, dadurch gekennzeichnet, daß Lactone gemäß Anspruch 1, bei denen der Rest A für

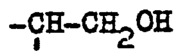

steht, auf Temperaturen zwischen 200 und 500°C erhitzt werden.

10. Verfahren zur Herstellung von Lactonen gemäß Anspruch 1 bei denen A für den Rest -CHY-CHY- steht, dadurch gekennzeichnet, daß man Lactone gemäß Anspruch 1, bei denen A für den Rest -CH=CH- steht, in einem inerten Lösungsmittel mit Chlor oder Brom umsetzt.

11. Verfahren gemäß Anspruch 10, dadurch gekennzeichnet, daß die Umsetzung bei Temperaturen zwischen -10 und +40°C durchgeführt wird.

12. Pentansäurederivate der Formel

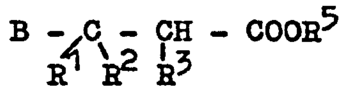

in der B für

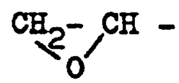,

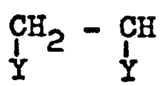 oder 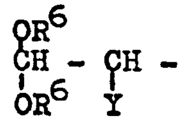 (Y = Cl oder Br).

und $R^5$ für einen niederen Alkylrest mit 1 bis 4 C-Atomen oder für ein Alkali- oder Erdalkalimetall oder für Wasserstoff steht und $R^1$, $R^2$ die im Anspruch 1 genannte Bedeutung haben und $R^3$ für einen $C_1$-$C_{10}$-Alkylrest steht und wobei mindestens einer der Reste $R^1$, $R^2$ oder $R^3$ mit Ausnahme für den Fall, daß

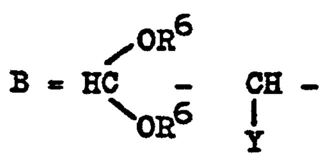

ist, ein solcher Alkylrest ist und $R^6$ einen Alkylrest mit 1 bis 10 C-Atomen bedeutet.

13. Verfahren zur Herstellung von Pentansäurederivaten gemäß Anspruch 12 bei denen B für den Rest

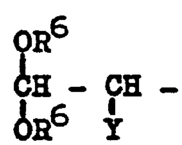

steht, dadurch gekennzeichnet, daß man eine Verbindung der Formel

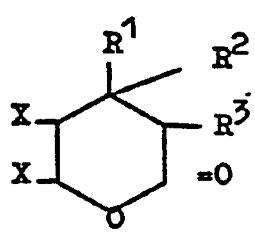,

in der $R^1$ und $R^2$ für Alkylreste mit 1 bis 10 C-Atomen und X ein Chlor- oder Bromatom darstellen, mit der 0,8- bis 1,2 fachen stöchiometrischen Menge eines Alkali- oder Erdalkalialkoholats in Gegenwart überstöchiometrischer Mengen von aliphatischen Alkoholen mit 1 bis 10 C-Atomen umsetzt.

14. Verfahren gemäß Anspruch 13, dadurch gekennzeichnet, daß man die Umsetzung bei Temperaturen zwischen -10 und +40°C durchführt.

15. Verwendung von ungesättigten Lactonen der Formel

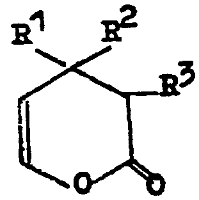

in der $R^1$ bis $R^3$ die in Anspruch 1 genannte Bedeutung haben $R^5$ für einen Alkylrest mit 1 bis 4 C-Atomen und $R^6$ für einen Alkylrest mit 1 bis 6 C-Atomen steht, zur Herstellung von Acetalen der 2,2-Dialkyl-3-formyl-cyclopropancarbonsäureester der Formel

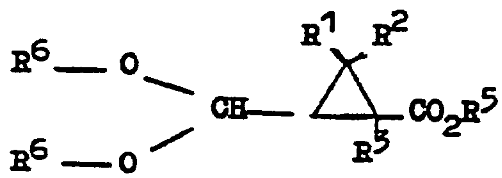

dadurch gekennzeichnet, daß man das ungesättigte Lacton mit Brom oder Chlor, gemäß Ansprüchen 10 und 11 reagieren läßt und anschließend das dabei erhaltene Dihalogenid mit Alkalialkoholaten umsetzt.

16. Verwendung der Lactone gemäß Anspruch 15, dadurch gekennzeichnet, daß man den bei der Umsetzung mit Alkalialkoholaten entstehenden Pentansäureester gemäß Anspruch 12 wobei B für

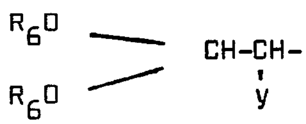 (y=Cl oder Br) steht,

(y = Cl oder Br) steht, nicht isoliert.

**Claims**

1. Substituted lactones of the formula

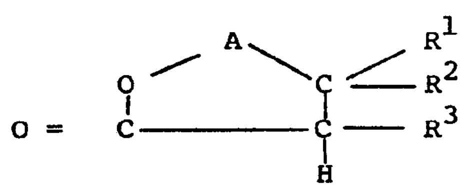

in which A stands for

$-CH-CH_2X$ (X=Cl, Br or OH),

-CHY-CHY-(Y=Cl or Br)

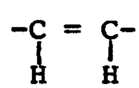

and $R^1$, $R^2$ and $R^3$ indicate the same or different residues from the group hydrogen and $C_1$ to $C_{10}$ - alkyl, with at least one of the residues $R^1$ or $R^2$ being such an alkyl residue.

2. Process for the preparation of lactones according to claim 1, in which the residue A stands for $-CH-CH_2Cl$

or -CH-$CH_2Br$, characterised in that pentenoic acid esters of the formula

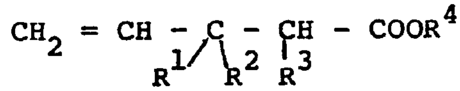

in which $R^1$, $R^2$ and $R^3$ have the above-indicated meaning are reacted with chlorine or bromine at temperatures between -10 and +40°C and the thereby obtained additional product is heated to temperatures between 150 and 200°C.

3. Process according to claim 2, characterised in that the addition reaction is carried out in liquid chlorohydrocarbon as solvent.

4. Process for the preparation of lactones according to claim 1, in which the residue A stands for -CH-$CH_2OH$, characterised in that lactones according to claim 1 in which the residue A is CH-$CH_2Br$ or -CH-$CH_2Cl$ are converted with basic means into pentenoic acid derivatives of the general formula

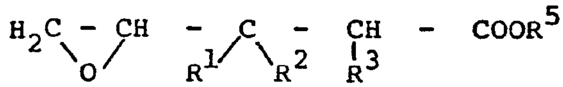

wherein $R^5$ stands for a lower alkyl residue with 1 to 4 C-atoms, hydrogen or for an alkali or alkaline earth metal, and then the lactone formation takes place by treatment with acids.

5. Process according to claim 4, characterised in that alkali or alkaline earth alcoholates of alcohols with 1 to 4 C-atoms are employed as basic means.

6. Process according to claim 4, characterised in that an alkali or alkaline earth hydroxide or carbonate is employed as basic means.

7. Process according to one of claims 4 to 6, characterised in that the lactone formation is carried out with the aid of mineral acids or organic acids.

8. Process according to one of claims 4 to 6, characterised in that the lactone formation is carried out with the aid of Lewis acids.

9. Process for the preparation of lactones according to claim 1, in which A stands for -CH=CH-, characterised in that lactones according to claim 1 in which the residue A stands for -CH-$CH_2OH$ are heated to temperatures between 200 and 500°C.

10. Process for the preparation of lactones according to claim 1, in which A stands for the residue -CHY-CHY-, characterised in that lactones according to claim 1 in which A stands for the residue -CH=CH- are reacted in an inert solvent with chlorine or bromine.

11. Process according to claim 10, characterised in that the reaction is carried out at temperatures between -10 and +40°C.

12. Pentanoic acid derivatives of the formulas

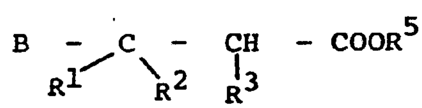

in which B stands for

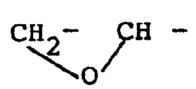 ,

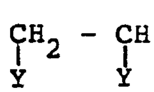 or 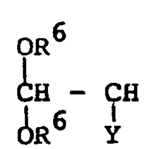 (Y = Cl or Br)

and $R^5$ stands for a lower alkyl residue with 1 to 4 C-atoms or for an alkali or alkaline earth metal or for hydrogen, and $R^1$, $R^2$ have the meaning indicated in claim 1 and $R^3$ stands for a $C_1$-$C_{10}$ alkyl residue, and with at least one of the residues $R^1$, $R^2$ or $R^3$, with the exception for the case that B is

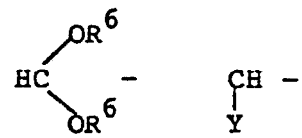

being such an alkyl residue and $R^6$ denoting an alkyl residue with 1 to 10 C-atoms.

13. Process for the preparation of pentanoic acid derivatives according to claim 12, in which B stands for the residue

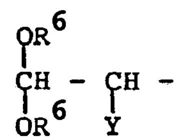

characterised in that a compound of the formula

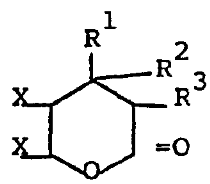

,

in which $R^1$ and $R^2$ stand for alkyl residues with 1 to 10 C-atoms and X stands for a chlorine or bromine atom, is reacted with the 0.8 to 1.2 times stoichiometric amount of an alkali or alkaline earth alcoholate in the presence of amounts in excess of stoichiometric of aliphatic alcohols with 1 to 10 C-atoms.

14. Process according to claim 13, characterised in that the reaction is carried out at temperatures between -10 and +40°C.

15. Use of unsaturated lactones of the formula

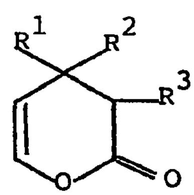

in which $R^1$ to $R^3$ have the meaning indicated in claim 1, $R^5$ stands for an alkyl residue with 1 to 4 C-atoms and $R^6$ for an alkyl residue with 1 to 6 C-atoms, for the preparation of acetals of 2,2-dialkyl-3-formyl-cyclopropane carboxylic acid esters of the formula

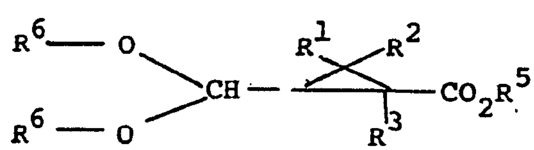

characterised in that the unsaturated lactone is allowed to react with bromine or chlorine according to claims 10 and 11 and then the dihalide thereby obtained is reacted with alkali alcoholates.

16. Use of the lactones according to claim 15, characterised in that pentanoic acid esters according to claim 12, wherein B stands for

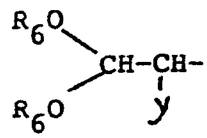 (y=Cl or Br)

being produced in the reaction with alkali alcoholates are not isolated.

**Revendications**

1. Lactones substituées de formule

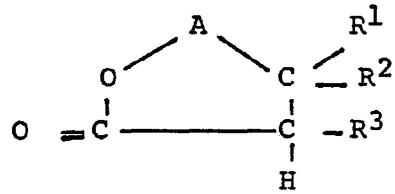

dans laquelle A représente

$-\overset{|}{C}H-CH_2X$ (X = Cl, Br ou OH),

$-CHY-CHY-$ (Y = Cl ou Br)

ou $-\underset{H}{\underset{|}{C}} = \underset{H}{\underset{|}{C}} -$

et $R^1$, $R^2$ et $R^3$ signifient des radicaux identiques ou différents du groupe de l'hydrogène et d'un alcoyle en $C_1$ à $C_{10}$, au moins un des radicaux $R^1$ ou $R^2$ étant ce radical alcoyle.

2. Procédé de fabrication de lactones selon la revendication 1 dans lesquelles le radical A représente

$-\underset{|}{C}H-CH_2Cl$ ou $-\underset{|}{C}H-CH_2Br$,

caractérisé en ce qu'on fait réagir un ester d'acide penténoïque de formule:

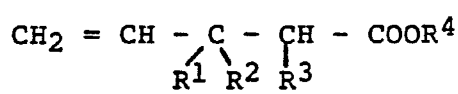

dans laquelle $R^1$, $R^2$ et $R^3$ ont la signification indiquée plus haut, avec du chlore ou du brome à des températures entre -10 et +40°C et en ce que l'on chauffe le produit d'addition ainsi obtenu à des températures entre 150 et 200°C.

3. Procédé selon la revendication 2, caractérisé en ce qu'on exécute la réaction d'addition dans un hydrocarbure chloré liquide comme solvant.

4. Procédé de fabrication de lactones selon la revendication 1 dans lesquelles le radical A représente

$-\underset{|}{C}H-CH_2OH$,

caractérisé en ce qu'on fait réagir des lactones selon la revendication 1 dans lesquelles le radical A est

$-CH-CH_2Br$

avec des agents basiques pour donner des dérivés d'acides pentanoïques de formule générale:

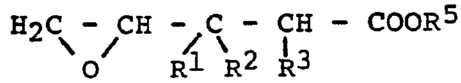

$R^5$ représentant un radical alcoyle inférieur ayant 1 à 4 atomes de carbone, de l'hydrogène ou un métal alcalin ou alcalino-terreux, et en ce qu'on exécute la formation de la lactone par traitement avec des acides.

5. Procédé selon la revendication 4, caractérisé en ce qu'on utilise comme agents basiques des alcoolates alcalins ou alcalino-terreux d'alcools ayant 1 à 4 atomes de carbone.

6. Procédé selon la revendication 4, caractérisé en ce qu'on utilise comme agent basique un hydroxyde ou carbonate alcalin ou alcalino-terreux.

7. Procédé selon l'une des revendications 4 à 6, caractérisé en ce qu'on exécute la formation de la lactone à l'aide d'acides minéraux ou d'acides organiques.

8. Procédé selon l'une des revendications 4 à 6, caractérisé en ce qu'on exécute la formation de la lactone à l'aide d'acides de Lewis.

9. Procédé de fabrication de lactones selon la revendication 1 dans lesquelles A représente -CH=CH-, caractérisé en ce qu'on chauffe à des températures entre 200 et 500°C des lactones selon la revendication 1 dans lesquelles le radical A représente

$$-CH-CH_2OH.$$

10. Procédé de fabrication de lactones selon la revendication 1 dans lesquelles A représente lé radical -CHY-CHY-, caractérisé en ce qu'on fait réagir dans un solvant inerte avec du chlore ou du brome des lactones selon la revendication 1 dans lesquelles A représente le radical -CH=CH-.

11. Procédé selon la revendication 10, caractérisé en ce qu'on exécute la réaction à des températures entre -10 et +40°C.

12. Dérivés d'acides pentanoïques de formule

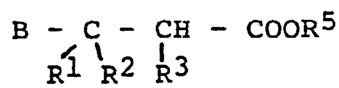

dans laquelle B représente

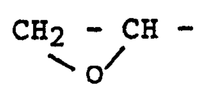 , 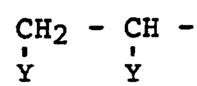

ou 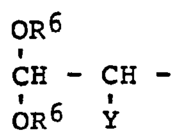 (Y = Cl ou Br)

et $R^5$ représente un radical alcoyle inférieur ayant 1 à 4 atomes de carbone, ou un métal alcalin ou alcalino-terreux, ou de l'hydrogène, $R^1$, $R^2$ ont la signification citée à la revendication 1 et $R^3$ représente un radical alcoyle en $C_1$ - $C_{10}$, au moins un des radicaux $R^1$, $R^2$ ou $R^3$, sauf dans le cas où B est

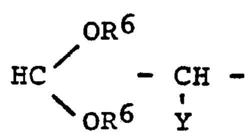 ,

signifiant ce radical alcoyle, $R^6$ etant un radical alcoyle ayant 1 à 10 atomes de carbone.

13. Procédé de fabrication de dérivés d'acides pentanoïques selon la revendication 12 dans lesquels B représente le radical

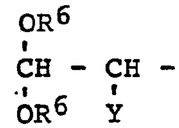

caractérisé en ce qu'on fait réagir un composé de formule:

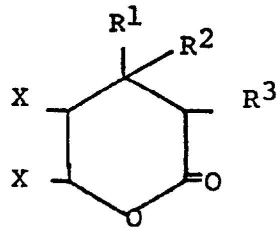

dans laquelle $R^1$ et $R^2$ représentent des radicaux alcoyle ayant 1 à 10 atomes de carbone et X un atome de chlore ou de brome, avec 0,8 à 1,2 fois la quantité stoechiométrique d'un alcoolate alcalin ou alcalino-terreux en présence de quantités supérieures à la stoechiométrie d'alcools aliphatiques ayant 1 à 10 atomes de carbone.

14. Procédé selon la revendication 13, caractérisé en ce qu'on exécute la réaction à des températures entre -10 et +40°C.

15. Utilisation de lactones insaturées de formule:

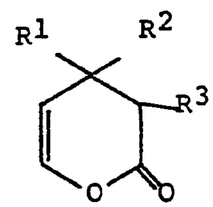

dans laquelle $R^1$ à $R^3$ ont la signification indiquée à la revendication 1, $R^5$ représente un radical alcoyle ayant 1 à 4 atomes de carbone et $R^6$ un radical alcoyle ayant 1 à 6 atomes de carbone, pour la fabrication d'acétals d'esters d'acides 2,2-dialcoyl-3-formyl-cyclopropanecarboxyliques de formule:

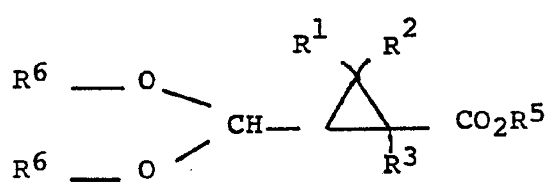

caractérisée en ce qu'on fait réagir la lactone insaturée avec du brome ou du chlore selon les revendications 10 et 11 puis on fait réagir le dihalogénure ainsi obtenu avec des alcoolates alcalins.

16. Utilisation des lactones selon la revendication 15, caractérisée en ce qu'on n'isole pas l'ester d'acide pentanoïque selon la revendication 12 obtenu dans la réaction avec des alcoolates alcalins, B représentant

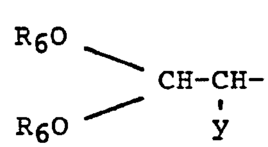

(y = Cl ou Br)